# EUROPEAN PATENT SPECIFICATION

(11) **EP 2 777 699 B1**
(45) Date of publication and mention of the grant of the patent: **29.03.2017**
(21) Application number: 12380005.4
(22) Date of filing: 02.02.2012
(51) Int. Cl.: A61K 31/185, A61P 27/02

(54) **Diethylamine-2,5-dihydroxybenzenesulfonate for the treatment of age-related macular degeneration**
Diethylamin-2,5-dihydroxybenzolsulfonat zur Behandlung von altersbedingter Makuladegeneration
2,5-Dihydroxybenzènesulfonate de diéthylamine pour le traitement de la dégénérescence maculaire liée à l'âge

(43) Date of publication of application: 17.09.2014
(73) Proprietor: INVESFOVEA S.L., 28008 Madrid (ES)
(72) Inventor: OUTEIRIÑO MIGUEZ, Luis Antonio, 28046 Madrid (ES)
(74) Representative: Toro Gordillo, Ignacio Maria

(56) References cited:
- WO-A1-2008/020032
- OLEJNIK O ET AL: "Drug delivery strategies to treat age-related macular degeneration", ADVANCED DRUG DELIVERY REVIEWS, ELSEVIER, AMSTERDAM, NL, vol. 57, no. 14, 13 December 2005 (2005-12-13), pages 1991-1993, XP027771383, ISSN: 0169-409X [retrieved on 2005-12-13]
- FEIGL ET AL: "Age-related maculopathy - Linking aetiology and pathophysiological changes to the ischaemia hypothesis", PROGRESS IN RETINAL AND EYE RESEARCH, OXFORD, GB, vol. 28, no. 1, January 2009 (2009-01), pages 63-86, XP025947156, ISSN: 1350-9462, DOI: 10.1016/J.PRETEYERES.2008.11.004 [retrieved on 2008-12-03]
- FOR THE CALDIRET STUDY GROUP ET AL: "Effect of calcium dobesilate on occurrence of diabetic macular oedema (CALDIRET study): randomised, double-blind, placebo-controlled, multicentre trial", THE LANCET, LANCET LIMITED. LONDON, GB, vol. 373, no. 9672, 18 April 2009 (2009-04-18), pages 1364-1371, XP026222191, ISSN: 0140-6736, DOI: 10.1016/S0140-6736(09)60218-X [retrieved on 2009-04-18]

## Description

### FIELD OF THE INVENTION

The invention describes the use of dobesilate and pharmaceuticals acceptable salts thereof for treating age-related macular degeneration in patients. In the invention the pharmaceutically acceptable salt is diethylamine

### BACKGROUND OF THE INVENTION

Age-related macular degeneration, as the term implies, affects older adults and accounts for about half of all vision impairment or blind registration in the developed world (Owen et al. Br J Ophthalmol 2003). Its prevalence is increasing with the global demographic shift towards an ageing society. The two threatening vision manifestations of age-related macular degeneration (AMD) occur either as a consequence of choroidal neovascularization (wet AMD), which causes an acute exudative pathology, or from cell loss due to geographic atrophy (dry AMD). In the first condition infiltrative edema produces very significant disturbance of retinal architecture layer causing a great disturbance of visual acuity. Drusen that are opaque yellowish white spots visible in the fundus oculi are the morphologic characteristic findings of dry AMD. In OCT images drusen appears as invagination (tears) into the sensorial retina between the retinal pigment epithelium (RPE) and Bruch membrane.

The main aims of treatment are either to prevent the progression of early disease or to treat the manifestations of advanced disease if these do occur. However, actual treatments for established wet AMD are associated in many patients with side effects such as inflammation and fibrosis (Tatar et al. Arch Ophthalmol 2000; Arevalo et al. Br J Ophthalmol 2008; Van Geest RJ et al. Br J Ophthalmol 2012; Jan 20 (Epub ahead of print) and also these therapies are very expensive. Furthermore, the gold standard treatment of wet AMD, chronically used was associated in 20% of patients with irreversible vision loss (Eldem B. Retina Congress Scientific Abstracts 2009; Chakravarthy U, Evans J, Rosenfeld. BMJ 2010). There is no effective treatment at the moment for dry AMD. Thus, the search of new efficient, safe and less expensive treatment modalities for AMD are urgently needed.

Inventors surprisingly found that dobesilate is capable to normalize retinal disturbed architecture and concomitantly increase visual acuity in patients affected for both forms of AMD. Described herein is the use of dobesilate and their derivatives, its pharmaceutically acceptable salts and solvates in the elaboration of a medicine for the treatment of dry and wet forms of AMD.

A first aspect of the invention relates to diethylamine-2,5-dihydroxybenzenesulfonate for use in the treatment of both forms of age-related macular degeneration.

These and other aspects of the present invention are explained in detail herein.

### ABSTRACT OF THE DISCLOSURE

The disclosure describes compositions and use of 2,5-dihydroxybenzene sulfonic acid and pharmaceutically acceptable salts thereof for treating age-related macular degeneration. In the invention the therapeutic agent is diethylamine 2,5-dihydroxybenzene sulfonate. The effect of visual acuity gain after dobesilate treatment might be related to the normalization of retinal architecture.

### BRIEF DESCRIPTION OF FIGURES

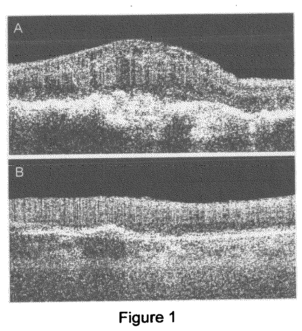

**Figure 1** Shows images of ocular coherence tomography from a patient with wet AMD before treatment (A) and after seven days of intravitreal application of dobesilate (B). After dobesilate treatment retinal macular thickness decreased significantly compared to baseline.

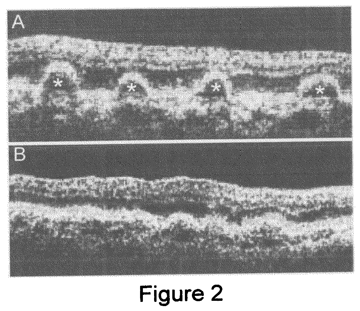

**Figure 2** Optical coherence tomograms from a patient with dry AMD before (A) and after (B) treatment with dobesilate. Note the presence of drusen tears (asterisks) in panel A. Treatment achieves normalization of retinal structure as consequence of disappearance of drusen tears.

### DETAILED DESCRIPTION OF THE INVENTION

As used throughout the disclosure, the following terms, unless otherwise indicated, shall be understood to have the following meanings.

"Patient" refers to animals, preferably mammals, most preferably humans, and includes males and females.

"Effective amount" refers to the amount of the compound and/or composition that is effective to achieve its intended purpose.

"Treating"; "treat" or "treatment" refers to using the compound or compositions of the present invention either prophylactically to prevent the symptoms of the disease or disorder, or therapeutically to ameliorate an existing condition.

"Therapeutic agent" includes any therapeutic agent can be used to treat or prevent the disease described herein. Therapeutic agents include, but are not limited to, antioxidants, anti-inflammatory compounds and the like. Therapeutic agent includes the pharmaceutically acceptable salts thereof, pro-drugs and pharmaceutical derivatives thereof.

"Transepithelial" refers to the delivery of a compound by application to the ocular surface.

"Intravitreal" refers to the delivery of a compound by application directly in the vitreous.

"Penetration enhancement" or "permeation enhancement" refers to an increase in the permeability of the epithelial tissue to a selected pharmacologically active compound such that the rate at which the compound permeates through the epithelial tissue is increased.

"Carriers" or "vehicles" refers to carrier materials suitable for compound administration and include any such material known in the art such as, for example, any liquid, gel, cream, solvent, liquid diluents, solubilizer or the like, which is non-toxic and which does not interact with any components of the composition in a deleterious manner.

"Sustained release" refers to the release of an active compound and/or composition that are maintained within a desirable therapeutic range over a period of time. The sustained release formulation can be prepared using any conventional method known to one-skilled in the art to obtain the desired release characteristics.

The compound used in this invention can be synthesized by one skilled in the art using conventional methods or is commercially available. The synthesis of these compounds is disclosed in for example "The Merck Index" 13th edition, Merck & Co, R. Rahway N.J. USA. 2001.

The compound and compositions for use according to the invention can be administered by any available and effective delivery system, including, but not limited to by topical application, subconjunctival application or by intravitreal administration in dosage unit formulations containing conventional non-toxic pharmaceutical acceptable carriers, adjuvant and vehicles as desired.

Topical administration of the compound and compositions for use according to the invention can be in the form of creams, gels, ointments, dispersions, solid sticks, emulsions, microemulsions and eye drops that can be formulated according to the conventional methods using suitable excipients.

Injectable preparations, for example, sterile injectable aqueous suspensions can be formulated according to the known act using suitable dispersing agents, wetting agents and/or suspending agents. The sterile injectable preparations can also be a sterile injectable solution or suspension in a non-toxic acceptable diluent or solvent. Among the acceptable vehicles and solvents that can be used are water, Ringer's solution, and isotonic sodium chloride solution.

Generally, the dosage requires to provide an effective amount of the compound and composition, which can be adjusted by one of ordinary skill in the art, will vary depending on the extent of the disease, frequency of treatment, the route of administration, pharmacological considerations such as the activity, efficacy, pharmacokinetic and toxicology profiles of the compound, whether a drug delivery system is used, and whether the compound is administered as part of a drug combination.

The amount of dobesilate that will be effective in the treatment of AMD, can be determined by standard clinical techniques, including reference to Goodman and Gilman supra: the Physician's Desk Reference, Medical Economics Company, Inc.; Oradell N.J. 1995; and Drug Facts and Comparisons; Inc. St. Louis, MO, 1993. The precise dose to be used in the formulation will also depend on the route of administration, and the seriousness of the disease or disorder, and should be decided by the physician and the patient's circumstances.

### EXAMPLES

The following non-limiting example further describes and enables one of ordinary skill in the art to make and use the present invention.

### Example 1. Effect of dobesilate in wet age-related macular degeneration

A 70-year-old man previously treated with intravitreal bevacizumab and ranibizumab with unsatisfactory results referred intense methamorphia in the left eye. Patient received an intravitreal solution of dobesilate (150µl) under sterile conditions, and following the International Guidelines for intravitreal injections (Aiello L. Retina 2004), in his left eye. Dobesilate was administered as a 12.5% solution of diethylmetylammonium 2,5-dihydroxybenzene sulfonate (Etamsylate, Dicynone^{®}. Sanofi-Aventis). The pH of the solution was 3.2 at the opening of the vial and 5.2 after a 1:20 dilution in Milli-Q^{®,} respectively. Ophthalmic evaluations included Shellen visual acuity (VA) testing and optical coherent tomography (OCT) through the dilated pupila. OTC is a powerful interferometric technology used to obtain cross sectional images of tissue non invasively with micrometer resolution, millimetre penetration depth and video-rate imaging speed (Huang Science 1991; 254: 1178-1181). This form of optical histology is ideal for imaging laminar structures such as the retina. For OCT studies we used a 30° horizontal line passing directly through the centre of the fovea. Central thickness was measured in OCT and defined as distance between the internal limiting membrane and retinal pigment epithelium (RPE) and they include intraretinal fluid.

At baseline, OCT shows significant increase of retinal thickness with an important retinal architecture perturbation (A). Normalization of retinal thickness was documented by OCT after dobesilate treatment at seven days follow-up (B) after intravitreal dobesilate injection, retinal macular thickness decreased significantly by 217 µm (530µm before treatment vs 312 µm after treatment). This normalization of retinal architecture after dobesilate treatment was associated with a corresponding visual improvement. Dobesilate increased a 25% of visual acuity (VA) after dobesilate treatment. Choroidal detail reveals in OCT pictures decreased vascular images, better noticed in the area of the fovea.

### Example 2- Effect of dobesilate in dry age-related macular degeneration

A 57-year-old man with dry-age related macular degeneration, previously treated with intravitreal bevacizumab (Avastin^{®}) with unsatisfactory results. Patient referred intense methamorphia in his right eye the last five days. Patient received an intravitreal solution of dobesilate (150µl) under sterile conditions, and following the International Guidelines for intravitreal injections (Aiello L. Retina 2004; 24:S9-S19 in his right eye. Dobesilate was administered as a 12.5% solution of diethylmetylammonium 2,5-dihydroxybenzene sulfonate (Etamsylate, Dicynone^{®}. Sanofi-Aventis). Ophthalmic evaluations included Shellen visual acuity (VA) testing and optical coherent tomography (OCT). Complete resolution of retinal structure disturbance was documented by OCT at eleven days follow-up (for comparison see optical coherence tomograms before A and B after treatment). Note that drusen images (A, asterisks) have disappeared after treatment (B). This normalization of retinal structure parallels with a corresponding visual improvement. Dobesilate doubled VA (0.4 at baseline vs 0.8 after treatment).

## Claims

1. Diethylamine-2,5-dihydroxybenzenesulfonate for use in the treatment of both forms of age-related macular degeneration.

2. Diethylamine-2,5-dihydroxybenzenesulfonate for use according to claim 1, wherein the therapeutic agent can be administered by topical application, subconjonctival application or intravitreal application.

3. Diethylamine-2,5-dihydroxybenzenesulfonate for use according to claim 1, wherein the therapeutic agent can be administered using a sustained release formulation of active compound.

4. Diethylamine-2,5-dihydroxybenzenesulfonate for use according to any of claims 1 to 3, wherein the therapeutic agent can be administered using a sterile injectable aqueous suspension.

5. Diethylamine-2,5-dihydroxybenzenesulfonate for use according to claim 1, to increase visual acuity associated with both forms of age-related macular degeneration.

## Patentansprüche

1. Diethylamin-2,5-dihydroxybenzensulfonat zur Anwendung in der Behandlung von beiden Formen der altersbedingten Makuladegeneration.

2. Diethylamin-2,5-dihydroxybenzensulfonat zur Anwendung nach Anspruch 1, wobei das therapeutische Mittel durch topische Anwendung, subkonjunktivale Anwendung oder intravitreale Anwendung verabreicht werden kann.

3. Diethylamin-2,5-dihydroxybenzensulfonat zur Anwendung nach Anspruch 1, wobei das therapeutische Mittel unter Verwendung einer Retardformulierung einer aktiven Verbindung verabreicht werden kann.

4. Diethylamin-2,5-dihydroxybenzensulfonat zur Anwendung nach einem der Ansprüche 1 bis 3, wobei das therapeutische Mittel unter Verwendung einer sterilen, injizierbaren wässrigen Suspension verabreicht werden kann.

5. Diethylamin-2,5-dihydroxybenzensulfonat zur Anwendung nach Anspruch 1, um die Sehschärfe in Verbindung mit den beiden Formen der altersbedingten Makuladegeneration zu erhöhen.

## Revendications

1. Le 2,5-dihydroxybenzènesulfonate de diéthylamine pour son utilisation dans le traitement des deux formes de dégénérescence maculaire liée à l'âge.

2. Le 2,5-dihydroxybenzènesulfonate de diéthylamine pour son utilisation selon la revendication 1, dans lequel l'agent thérapeutique peut être administré par application topique, application sous-conjonctivale ou application intravitréale.

3. Le 2,5-dihydroxybenzènesulfonate de diéthylamine pour son utilisation selon la revendication 1, dans lequel l'agent thérapeutique peut être administré en utilisant une formulation à libération prolongée de composé actif.

4. Le 2,5-dihydroxybenzènesulfonate de diéthylamine pour son utilisation selon l'une quelconque des revendications 1 à 3, dans lequel l'agent thérapeutique peut être administré en utilisant une suspension aqueuse injectable stérile.

5. Le 2,5-dihydroxybenzènesulfonate de diéthylamine pour son utilisation selon la revendication 1, pour augmenter l'acuité visuelle associée aux deux formes de dégénérescence maculaire liée à l'âge.
